# FASCICULE DE BREVET EUROPEEN

(11) **EP 2 699 160 B1**
(45) Date de publication et mention de la délivrance du brevet: **18.03.2015**
(21) Numéro de dépôt: 12728626.8
(22) Date de dépôt: 20.04.2012
(51) Int. Cl.: A61B 5/11, A61G 13/00

(54) **APPAREIL DE DETECTION D'UNE LESION PARTIELLE DE LIGAMENT CROISE ANTERIEUR DU GENOU**
VORRICHTUNG ZUM NACHWEISEN EINER PARTIELLEN LÄSION DES VORDEREN KREUZBANDES
APPARATUS FOR DETECTING A PARTIAL LESION OF THE ANTERIOR CRUCIATE LIGAMENT OF THE KNEE

(30) Priorité: 22.04.2011 FR 1101285
(43) Date de publication de la demande: 26.02.2014
(73) Titulaire: GENOUROB, 53000 Laval (FR)
(72) Inventeur: ROBERT, Henri, F-53100 Mayenne (FR); NOUVEAU, Stephane, F-53970 L'Huisserie (FR)
(74) Mandataire: Benech, Frédéric
(86) Numéro de dépôt international: PCT/FR2012/000157
(87) Numéro de publication internationale: WO 2012/143628

(56) Documents cités:
- JP-A- 9 276 252
- ROBERT H ET AL: "A new knee arthrometer, the GNRB<(>R): Experience in ACL complete and partial tears", ORTHOPAEDICS & TRAUMATOLOGY: SURGERY & RESEARCH,, vol. 95, no. 3, 1 mai 2009 (2009-05-01), pages 171-176, XP026146862, ISSN: 1877-0568, DOI: 10.1016/J.OTSR.2009.03.009 [extrait le 2009-05-07]
- JUNG HO-JOONG ET AL: "Role of biomechanics in the understanding of normal, injured, and healing ligaments and tendons", SPORTS MEDICINE, ARTHROSCOPY, REHABILITATION, THERAPY & TECHNOLOGY, BIOMED CENTRAL LTD, vol. 1, no. 1, 20 mai 2009 (2009-05-20), page 9, XP021061051, ISSN: 1758-2555, DOI: 10.1186/1758-2555-1-9
- TSAI ANDREW G ET AL: "Rotational knee laxity: Reliability of a simple measurement device in vivo", BMC MUSCULOSKELETAL DISORDERS, BIOMED CENTRAL, LONDON, GB, vol. 9, no. 1, 18 mars 2008 (2008-03-18), page 35, XP021035539, ISSN: 1471-2474
- GABRIEL M T ET AL: "Distribution of in situ forces in the anterior cruciate ligament in response to rotatory loads", JOURNAL OF ORTHOPAEDIC RESEARCH, JOHN WILEY & SONS, INC, NEEDHAM, MA, vol. 22, no. 1, 1 janvier 2004 (2004-01-01), pages 85-89, XP004722555, ISSN: 0736-0266, DOI: 10.1016/S0736-0266(03)00133-5
- WOO SAVIO L-Y ET AL: "Biomechanics and anterior cruciate ligament reconstruction", JOURNAL OF ORTHOPAEDIC SURGERY AND RESEARCH, BIOMED CENTRAL LTD, LO, vol. 1, no. 1, 25 septembre 2006 (2006-09-25), page 2, XP021020336, ISSN: 1749-799X, DOI: 10.1186/1749-799X-1-2

## Description

La présente invention concerne un appareil de mesure de lésion des ligaments du genou.

Elle trouve une application particulièrement importante, bien que non exclusive, dans le domaine de la détection d'une lésion partielle du ligament croisé antérieur (LCA) d'un genou de patient.

On connaît déjà (US 4,534,364) un dispositif permettant de mesurer les déplacements d'un genou afin de déterminer et de traiter une insuffisance ligamenteuse.

Pour ce faire deux jambes du patient sont placées sur un support en forme de T réglable, présentant un angle déterminé avec les cuisses et on applique manuellement une force en tirer/pousser pour mesurer le mouvement relatif entre tibia et fémur, grâce au déplacement d'un indicateur gradué qui reste en position par frottement, une fois les déplacements réalisés.

Un tel dispositif, d'une part ne permet pas de mesures précises, et d'autre part n'est pas répétitif, du fait notamment des perturbations nées du comportement même du patient (crispation incontrôlée des muscles faussant les mesures, torsions des jambes liées à l'angle d'inclinaison de la table introduisant des contraintes parasites, etc...) .

On connaît également (WO 88/04536) un dispositif d'analyse du genou dans lequel on fixe les condyles tibia et fémoral, on fixe le pied du patient correspondant et on applique un couple de façon incrémentale pour mesurer en continu les charges et couples appliqués, pour en déduire la stabilité du genou concerné.

Des systèmes de cordes et de poulies permettent la mise en place de telles charges.

Outre le fait qu'un tel dispositif est compliqué, il ne tient pas compte de la rotation du pied lors de la flexion du genou et ne permet pas de détecter de façon fine comment il peut y avoir lésion et de quelle lésion il s'agit.

On connaît aussi (WO 2008/040790) un dispositif de détection et de suivi d'une lésion du ligament croisé antérieur du genou comportant un support de la jambe muni d'une part de moyen de poussée de la face postéro-supérieure du mollet et d'autre part d'un capteur de déplacement du tibia.

Un tel dispositif qui permet d'obtenir des résultats intéressants peut encore être amélioré. Il est aussi connu (JP 9 276252 A) un dispositif selon le préambule de la revendication 1.

Tous ces dispositifs connus présentent en effet et notamment l'inconvénient de ne pas permettre clairement et de façon répétitive la détection d'une lésion partielle d'un ligament.

En effet s'il existe des dispositifs connus permettant de détecter de façon assez fiable une rupture totale des ligaments, le dépistage des lésions partielles, en particulier celles du LCA n'est pas aujourd'hui effectué de façon satisfaisante.

On sait que le ligament croisé antérieur (LCA) est composé de très nombreuses fibres tendineuses que l'on peut séparer en deux groupes ou faisceaux selon leur insertion sur le tibia et le fémur, à savoir un faisceau antéro-médial (AM) et un faisceau postéro-latéral (PL).

Or, en fonction des traumatismes subis par le patient, ces ruptures du LCA peuvent être totales ou incomplètes (rupture d'un faisceau, rupture avec cicatrisation ectopique, distension globale...).

Les ruptures d'un faisceau sont ainsi habituellement dénommées des ruptures partielles AM ou PL. Leurs dépistages restent difficiles sur le plan clinique, et radiologique malgré les progrès de l'IRM. Seule l'arthroscopie permet en fait en dernier ressort de préciser le type de rupture et son siège.

Mais une telle analyse est coûteuse et nécessite du temps.

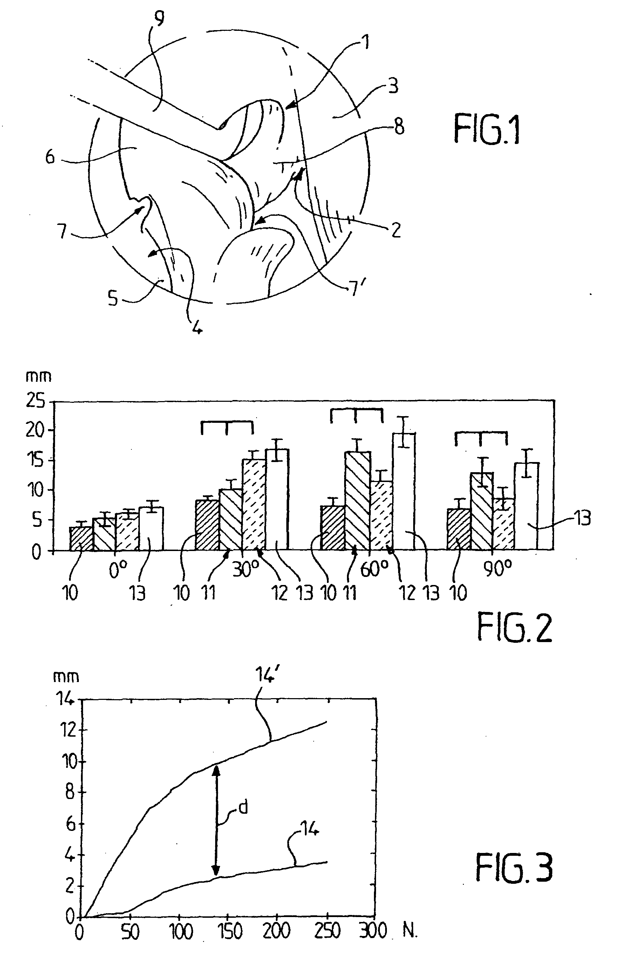

On a représenté sur la figure 1 une vue schématique d'un exemple de l'image d'un ligament croisé antérieur 1 partiellement rompu.

Le ligament 1 est fixé d'un côté 2 sur le tibia 3 et de l'autre côté 4 sur le fémur 5.

Le ligament 1 comprend le faisceau AM 6 en partie déchiré en 7 et 7', au premier plan, et le faisceau PL 8, en bon état, à l'arrière plan. Les deux faisceaux sont séparés l'un de l'autre, afin d'illustration, par un outil 9 sur la figure.

Il est tout à fait crucial de pouvoir élaborer parfois assez rapidement un diagnostic précis de la lésion du patient.

En effet par exemple pour un sportif dont la solidité des genoux conditionne ses performances et sa participation ou son maintien dans un match, une lésion partielle peut ne pas être détectée correctement à chaud. Alors que l'arrêt de l'utilisation d'un genou et une convalescence de quelques jours peuvent permettre à la lésion partielle de se réparer, celle-ci si elle n'est pas proprement détectée, peut devenir totale, ce qui présente des conséquences parfois très graves.

La présente invention vise à fournir un appareil de détection d'une lésion partielle du genou répondant mieux que ceux antérieurement connus aux exigences de la pratique, notamment en ce qu'elle permet la détection d'une lésion ou rupture partielle du LCA de façon rapide et fiable, en dynamique, ainsi que l'identification de l'emplacement d'une telle rupture (AM ou PL).

Grâce à l'invention, un début de rupture peut être diagnostiqué très tôt, ce qui permet de laisser le temps au patient de cicatriser plutôt que de risquer la rupture définitive.

L'intérêt de la détection précoce est aussi de permettre la mise en place d'une rééducation adaptée pour protéger le ligament pendant cette phase de cicatrisation.

Pour ce faire l'invention part notamment de l'idée de mesurer successivement et en dynamique le comportement de l'articulation du genou du patient à différents niveaux de flexion du genou et ce tout en supprimant les contraintes autres que celles correspondantes à une sollicitation directe de l'articulation.

Une telle méthode repose notamment sur l'observation suivante :

Lorsqu'on exerce une pression sous le mollet, (générant une translation antérieure du tibia), tout comme lorsqu'on impose une flexion du genou avec un angle compris entre 10° et 70°, on induit un mouvement de rotation du tibia et donc du pied, qu'il convient de rendre libre pour ne pas ajouter une contrainte perturbant la mesure.

L'invention est également basée sur les mesures effectuées lors d'une étude de laboratoire (cf. figure 2) mesurant la translation antérieure du tibia soumis à des poussées effectuées jusqu'à 134 newtons, à plusieurs angles de flexion, en cas de lésion de l'un et/ou de l'autre des faisceaux AM et PL.

Plus précisément on a représenté sur la figure 2 en abscisses le degré de flexion du genou, et en ordonnées le mouvement de translation antérieure du tibia en mm, mesuré avec un capteur du type de celui qui sera détaillé en référence au mode de réalisation plus particulièrement décrit ci-après.

Les mesures correspondantes à un genou intact sont montrées sur les colonnes 10, celles correspondantes à une lésion du faisceau AM en colonnes 11, celles correspondantes à un défaut du faisceau PL en colonnes 12 et celles correspondantes à une rupture totale du ligament en colonnes 13.

On constate qu'à 30° la translation est plus importante en cas de lésion (ici section totale) du faisceau PL qu'en cas de lésion du faisceau AM, et que l'inverse se produit à 60°.

L'invention va donc utiliser ces modifications de laxité différentielles (genou sain versus genou pathologique) entre 30° et 60° pour localiser la lésion.

En d'autres termes, si le différentiel augmente en flexion de 0° à 60°, le faisceau AM ne remplit plus son rôle de frein, ce qui démontre qu'il est lésé, et inversement pour le faisceau PL.

Dans le but ci-dessus et pour permettre de palier les inconvénients de l'art antérieur, la présente invention propose notamment un appareil selon la revendication 1.

Rappelons qu'un corps peut se déplacer selon six degrés de liberté, à savoir trois en translation et trois en rotation autour d'axes fixes dans les trois directions d'un repère orthonormé.

Avec le mode de réalisation de l'invention plus particulièrement décrit, et grâce à la mobilité du pied et à la possibilité de mouvement relatif entre assise et support en cas de flexion, aucune contrainte parasite n'est de ce fait générée, ce qui autorise la mesure fine de la lésion partielle.

Dans des modes de réalisation particuliers, on a de plus recours à l'une et/ou à l'autre des dispositions suivantes :
- Le support du pied est monté libre en translation dans le sens transversal par rapport au plateau support, sa position étant fixe et réglable pour les deux autres degrés de liberté en translation, à savoir en hauteur et dans le sens longitudinal du dit support ;
- le support du pied comporte des moyens capteurs d'au moins un angle de rotation dudit support et les moyens de calcul sont agencés pour mesurer le mouvement de rotation enregistré par ledit capteur induit par le tibia lors de la flexion du plateau support par rapport à l'assise et/ou généré par la translation antérieure du tibia, pour en déduire la lésion partielle du ligament AM et/ou PL.

Rappelons ici les rôles respectifs des faisceaux AM et PL. Le faisceau AM contrôle la translation antérieure du genou, le faisceau PL freinant quant à lui la rotation interne, jouant de ce fait un rôle important dans l'instabilité rotatoire.
L'ajout des mesures de rotation va donc permettre de compléter efficacement la mesure de lésion partielle, en améliorant de façon importante et surprenante la précision et la prédictibilité L'angle de rotation mesuré est plus particulièrement celui dans le sens transversal par rapport au tibia, et/ou complété par l'angle dans le plan longitudinal, vers l'avant ou l'arrière du patient ;
- l'appareil comporte de plus au moins un capteur/détecteur de la contraction des muscles de la cuisse, et des moyens de traitement pour empêcher la prise en compte des mesures de déplacements et/ou de force de poussée tant que ladite contraction musculaire n'est pas descendue en dessous d'un seuil déterminé ;
- le capteur de détection de déplacement est fixé de façon réglable en hauteur à une potence de support du capteur solidaire du plateau support de la jambe ;
- l'organe de poussée comprend un coussin de soutien du mollet fixé rigidement à une biellette reliée de façon articulée à un système de vérin axial monté parallèle au plateau support et propre à actionner perpendiculairement à sa course ladite biellette articulée, entre une position de repos où la jambe repose librement sur le plateau support et une position où la force exercée sur le mollet atteint une valeur comprise entre 0 et 300 N lorsque la jambe est fixée au support.

De façon à exercer cette force la course en hauteur du coussin entre une position d'affleurement du mollet et une position haute maximale est par exemple comprise entre 0 et 15 mm, voir entre 0 et 20 mm.
- les moyens de fixation de la jambe au support comportent une coque de maintien quadricipital agencée pour être en contact avec le dessus de l'extrémité de la cuisse, rattachée au plateau support par un jeu de deux sangles parallèles semi-rigides réglables en serrage.

L'invention sera mieux comprise à la lecture de la description qui suit, de modes de réalisation donnés ci-après à titre d'exemples non limitatifs.

L'invention se réfère aux dessins qui l'accompagnent dans lesquels :
Les figures 1 et 2 déjà décrites, montrent pour l'une une vue schématique d'un ligament croisé antérieur partiellement rompu et pour l'autre les résultats d'une étude de laboratoire faisant apparaître la translation antérieure d'un tibia soumis à des poussées différentielles.
Les figures 3 et 4 donnent des exemples de courbes permettant la comparaison entre un genou sain et un genou pathologique, dans le cas d'une rupture totale (figure 3) ou dans le cas d'une rupture partielle (figure 4), obtenue avec un appareil selon l'invention.
La figure 5 est une vue générale partielle, en perspective, d'un appareil de mesure appliqué à un patient, selon le mode de réalisation de l'invention plus particulièrement décrit ici.
La figure 5A est une vue schématique de l'appareil de la figure 5.
La figure 6 est une vue latérale du plateau support de la jambe et de ses accessoires, selon le mode de réalisation de l'invention plus particulièrement décrit ici.
Les figures 7 et 7A montrent le support du pied du patient en vue latérale (figure 7) et en perspective (figure 7A) selon un mode de réalisation de l'invention.
La figure 8 est un organigramme montrant les étapes principales de la méthode selon un mode de réalisation de l'invention.

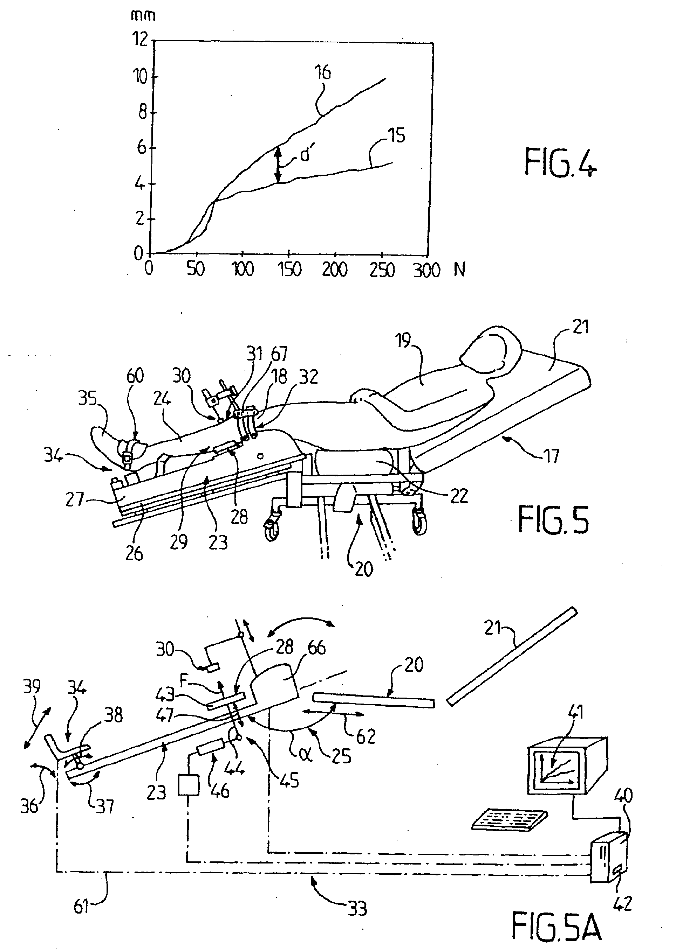

Les figures 3 et 4 montrent respectivement les courbes obtenues pour l'une et l'autre des jambes de deux patients différents. Elles donnent en ordonnée le déplacement du capteur de détection de la face antéro-supérieure du tibia, en mm, en fonction de la force de poussée en Newton exercée par l'organe de poussée du mollet, indiquée en abscisse.

La figure 3 est un exemple de résultats obtenus dans le cas d'une rupture complète des ligaments, et la figure 4 dans le cas d'une rupture partielle des ligaments et plus particulièrement et en l'espèce du faisceau antéro-médial (AM).

Plus précisément la figure 3 montre à titre d'exemple sur la courbe 14 la réaction d'un genou normal et sur la courbe 14' la réaction d'un genou ayant subi une rupture complète de son ligament croisé antérieur.

La différentielle de mesure d, qui correspond ici à 6 mm, va permettre de diagnostiquer la rupture.

En effet en règle générale on peut confirmer une rupture complète lorsque la translation différentielle du tibia est d'au moins 3 mm pour une force exercée de l'ordre de 134 N.

Sur la figure 4 on a représenté une rupture partielle du ligament.

La courbe 15 correspond au comportement normal d'un ligament croisé antérieur de genou et la courbe 16 à celui d'un ligament partiellement abimé.

On constate ici (dans l'exemple proposé) que le différentiel d' à 134 N est de 2,5 mm. Il en résulte que, si on se contente d'effectuer une comparaison du différentiel, on peut se retrouver en dessous du seuil de détection alors qu'en fait il y a un problème.

La présente invention vise justement à permettre, malgré cette comparaison qui ne devrait pourtant pas autoriser le diagnostic de rupture, de diagnostiquer une telle rupture partielle, grâce notamment à la pluralité de mesures effectuées dans différentes inclinaisons du support.

Les figures 5 et 5A montrent un appareil de mesure 17 pour détection d'une lésion du ligament croisé antérieur du genou 18 d'un patient 19. L'appareil 17 comprend une assise 20 du patient munie d'un dossier 21 orientable en inclinaison par rapport à l'assise. Celle-ci est par exemple constituée par un coussin 22 sur lequel le fessier du patient 19 repose.

L'appareil 17 comprend un plateau 23 de support de la jambe 24 du patient mobile en rotation (flèche 25 sur la figure 5A) par rapport à l'assise 20.

Les moyens pour actionner, régler (hauteur etc...) et/ou supporter l'assise, le dossier et le support sont connus en eux-mêmes et ne seront pas détaillés.

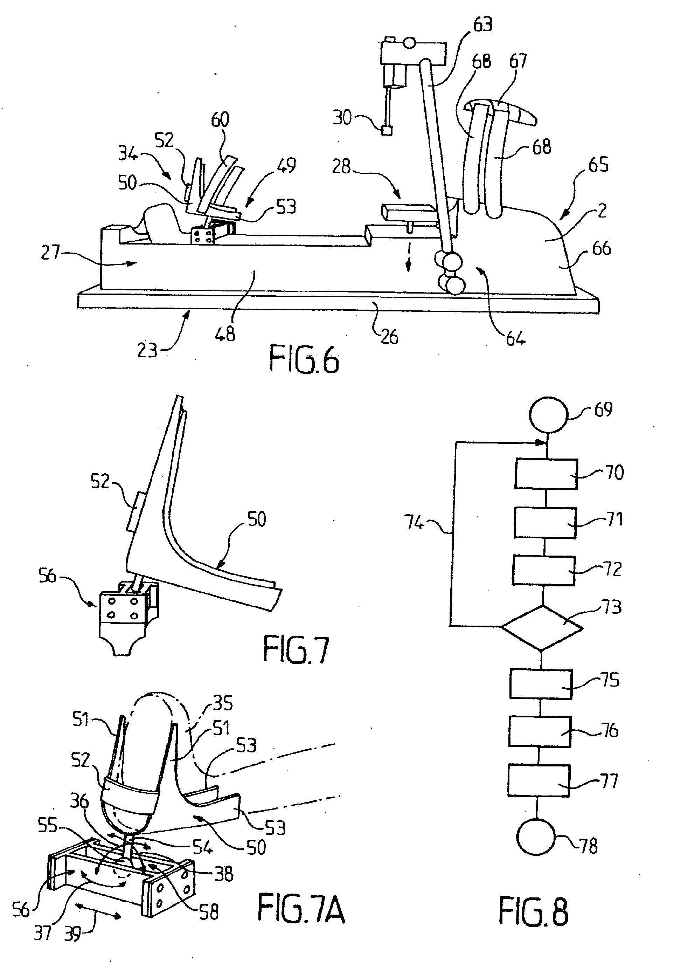

Le plateau 23 est par exemple formé par une plaque métallique rectangulaire 26 sur.laquelle est fixée une coque 27 de forme générale demi-cylindrique, de support de la jambe, et qui sera décrite plus avant en référence à la figure 6.

Il est par ailleurs muni d'un organe de poussée 28 du mollet 29 du patient et d'un capteur 30 de détection de déplacement de la face antério-supérieure 31 du tibia du patient lorsque le patient est assis sur l'assise.

Il est prévu des moyens de fixation 32 de la jambe audit support et (cf. figure 5A) des moyens 33 de mesure en continu du déplacement du capteur 30 en fonction de la force F exercée par l'organe de poussée 28 sur le mollet.

L'appareil 17 comprend également un support 34 du pied 35 du patient.

Le support 34 du pied est librement mobile dans les trois degrés de liberté 36, 37 et 38 en rotation (par rapport aux axes du repère orthonormé du pied, l'axe Ox étant l'axe longitudinal de la jambe). Il est également mobile en translation latérale comme cela sera indiqué plus précisément en référence aux figures 7 et 7A (flèche référencée 39-axe Oy).

L'appareil comporte des moyens d'enregistrement 40 des courbes 41 obtenues pour l'une et l'autre des jambes et donnant le déplacement du capteur en fonction de la force de poussée (cf. figure 3 et 4) mais dans des inclinaisons différentes (angles α-cf. flèche 25 sur la figure 5A) entre le support 23 et l'assise 20, et des moyens de calcul 42 agencés pour mesurer le différentiel entre ces courbes et en déduire la lésion partielle du ligament AM et/ou du ligament PL.

Les moyens d'enregistrement 40 sont par exemple et de façon connue en elle-même formés par une carte d'acquisition des valeurs transmises par le capteur 30, (qui peut par exemple être un capteur à potentiel), qui sont corrélés avec les valeurs de la force de poussée F exercée, ces valeurs faisant également partie des entrées de données avec celles des angles α d'inclinaison.

Toutes ces valeurs sont transmises aux moyens de calculs 42.

Ceux-ci comportent un microprocesseur d'ordinateur classique, programmé de façon comme en elle-même par l'homme du métier pour effectuer les fonctions de comparaison des courbes, en intégrant d'une part l'évolution du déplacement en fonction de l'angle (cf. figure 2), d'autre part l'inclinaison entre les courbes 15 et 16 (cf. figure 4)(le fait que cela soit un angle important et non des courbes parallèles) et enfin la valeur mesurée de d' proprement dite.

Les étapes mises en oeuvre dans cette comparaison arrivant à un diagnostic seront détaillées ci-après.

L'organe de poussée 28 comprend un coussin 43 de soutien du mollet fixé rigidement à une biellette 44 reliée de façon articulée en 45 à un système de vérin axial 46, parallèle au support, et propre à actionner perpendiculairement à sa course (flèche 47) la biellette 44 entre une position de repos où la jambe repose librement sur le support et une position où la force exercée sur le mollet atteint au moins 350 N, et de préférence jusqu'à 700 N par exemple 500 N.

On a utilisé dans la suite de la description les mêmes numéros de références pour désigner des mêmes éléments ou des éléments semblables.

La figure 6 montre le plateau support 23 des figures 5 et 5A, qui comporte la plaque 26 sur laquelle est fixé l'élément en forme de coque 27.

Celui-ci comprend une partie allongée 48 de support de l'essentiel de la jambe, terminé en 49 par le support 34 du pied.

Le support 34 (cf. également figure 7 et 7A) comprend une coque 50 de maintien du pied 35 sensiblement en forme de cornière évidée munie de deux parois latérales 51 reliées l'une à l'autre au niveau du talon du pied par une entretoise 52 d'appui dudit talon et par deux joues 53 de soutien de la cheville.

Ce support 34 comprend une tige verticale 54 cylindrique fixée à son extrémité à une boule sphérique 55 mobile en rotation selon les trois degrés de liberté (36, 37 et 38) dans un guide 56 formé par une pièce parallélépipédique définissant un évidement ou rainure 58 dans laquelle la sphère est retenue, par exemple par des lèvres supérieures longitudinales, rainures avec laquelle la boule sphérique 55 coopère à glissement autorisant la rotation libre du support de pied en rotation, comme décrit ci-avant. Une telle rainure permet également le mouvement en translation latérale (flèche 39).

De telles dispositions particulières permettent une mobilité complète sans aucune contrainte de la cheville et du pied du patient (et donc du tibia), comme cela sera décrit plus précisément ci-après.

La pièce parallélépipédique 56 est quant à elle déplaçable en hauteur, et longitudinalement, le long du support 23, en fonction de la longueur et de l'épaisseur de la jambe du patient à tester.

Ces déplacements se font à l'aide de moyens connus en eux-mêmes, par exemple par le biais d'une crémaillère à mollette et/ou actionnée par des vérins.

Enfin il est prévu des sangles 60, par exemple deux sangles parallèles, de maintien du pied dans le support 34, permettant de fixer l'ensemble pour autoriser un test de façon répétitive et fiable.

Selon le mode de réalisation de l'invention plus particulièrement décrit, le support du pied 34 comporte également des moyens capteurs connus en eux-mêmes, par exemple constitués par une inclinomètre du type par exemple capacitif (non représenté) d'au moins un angle de rotation dudit support connectés (trait mixte 61 de la figure 5A) au moyen de calcul et agencés pour mesurer le mouvement de rotation enregistré par ledit capteur induit par le tibia lors de la flexion du support par rapport à l'assise et l'intégrer s'il y a lieu aux éléments dores et déjà obtenus et pris en compte dans l'analyse de la lésion.

En effet les degrés de liberté en rotation du support du pied sont nécessaires pour libérer les tensions en rotation sur le tibia, lors de la mise en compression du mollet. Ils sont également utiles lors de la flexion différentielle de l'assise par rapport au support.

Plus précisément les moyens de mesure d'au moins un angle de rotation, ou d'inclinaison, par rapport aux axes du repère orthonormé attaché au pied, sont par exemple basés sur un accéléromètre trois axes relié à l'ordinateur 40 par exemple par une liaison de type USB.

Ces moyens permettent une mesure de rotation relative à la position verticale ou à une autre orientation.

La rotation relative mesurée est alors et par exemple la combinaison de la rotation autour des deux axes d'un repère 2D parallèle au sol (tangage et roulis), ou en d'autres termes la combinaison de deux degrés de liberté en rotation.

La rotation lacet autour du troisième axe n'est quant-à-elle pas prise en compte.

En d'autres termes, au fur et à mesure que cette assise bouge, le support va entraîner un mouvement de rotation du tibia dont on s'affranchit par l'intermédiaire de la libre orientation mentionnée ci-dessus, mais qui permet d'affiner la détection et l'ampleur de la lésion partielle.

Lors de la mise en flexion, l'assise se déplace par ailleurs suivant la flèche transversale 62 (sur la figure 5A) qui permet ici encore d'équilibrer les forces de tension sur le corps du patient et évite à celui-ci d'engendrer des contraintes risquant de modifier les conditions de mesure de la lésion.

Dans un mode de réalisation particulier, il est également prévu (non représentés) des moyens de mesure permettant de détecter toute contraction musculaire parasite qui empêche de déterminer avec exactitude le déplacement antérieur du tibia dû à la lésion.

Ceux-ci sont constitués de manière connue en elle-même, par exemple par un capteur/détecteur électrique de l'activité des muscles. Ce capteur est relié au moyen de calcul et agencé de façon connue pour, par une programmation adéquate, empêcher la prise en compte des mesures de déplacement et/ou des forces de poussée, tant que la contraction musculaire n'est pas descendue en dessous d'un seuil déterminé, correspondant à une décontraction musculaire suffisante, par exemple un signal électrique nul.

Le plateau de support de la jambe 23 comporte comme on l'a vu, et par ailleurs, le capteur 30 de détection de déplacement. Celui-ci est fixé de façon réglable en hauteur par une tige 63, au plateau 64 par exemple de façon articulée (64).

Le support 23 comprend par ailleurs à son extrémité 65, située du côté de l'assise du patient, une partie 66 en forme de coque bombée, arrondie et agencée pour supporter le creux de la cuisse un peu avant le genou.

Les moyens de fixation sur la jambe comprennent quant à eux une pastille en forme de calotte sphérique, ou coque 67 de maintien quadricipital, agencée pour être en contact avec le dessus de l'extrémité de la cuisse et rattachée au support par un jeu de deux sangles 68 parallèles semi-rigides réglables en serrage, par exemple en matière plastique.

Grâce à un tel jeu de sangles il est possible d'obtenir une excellente fixation de la jambe avec néanmoins la souplesse suffisante pour ne pas incommoder le patient en entrainant des réactions musculaires risquant de fausser la mesure, tout en autorisant une certaine souplesse dans les déplacements latéraux et transversaux de la jambe, au moment de sa mise en place.

Le capteur 30 de déplacement est par exemple formé par un capteur de déplacement linéaire avec ressorts de rappel de 10 à 100 mm fabriqués par la société française NOVOTECHNIK sous les références TR et/ou TRS, avec double paliers pour meilleur guidage.

On va maintenant décrire en référence aux figures 8 et 5A le fonctionnement de l'appareil 1.

Grâce à cet appareil il est possible de mesurer la détection d'une lésion de ligament croisé antérieur du genou d'un patient en cas de lésion partielle.

On sait que le diagnostic des ruptures d'un seul faisceau (antéro-médial ou postéro-latéral) du ligament croisé antérieur est cliniquement difficile, l'IRM n'étant pas toujours contributif.

Souvent la décision n'est alors prise que lors de l'arthroscopie. Grâce à l'invention utilisée en mode préopératoire, les courbes d'élasticité du LCA de chaque genou sont mesurées et on peut en les comparant détecter de quelle lésion il s'agit et ainsi programmer le type de réparation, complet ou partiel, ainsi que le traitement (opération ou convalescence) à préconiser en fonction des données préopératoires recueillies.

Pour ce faire et comme on l'a indiqué on enregistre la courbe d'élasticité de chaque LCA soumis à un effort de poussée compris entre 0 et 300 N.

L'analyse des courbes permet d'établir un différentiel de laxité par exemple à 134 N en mm et un différentiel des pentes en µm/N comme cela apparaît sur les figures 3 et 4.

L'expérience a ainsi montré que soixante-quinze ruptures unilatérales du LCA mesurées en préopératoire puis opérées d'une plastie avec un tendon ischio-jambier, avaient démontré que les informations recueillies grâce à l'invention sur le différentiel de laxité à 134 N combinés aux pentes mesurées entre les deux genoux obtenues, ainsi que en faisant varier les angles d'inclinaison, les mesures étant effectuées à 30°, 45° et 60° avaient permis une grande prédictibilité du type de lésion.

Selon les tests et les constatations opératoires, une réparation partielle (quinze cas) ou complète (cinquante-cinq cas) a été réalisée.

Ainsi un seuil de laxité différentiel < à 3 mm à 134 N permet le diagnostic préopératoire de réparation d'un faisceau avec une sensibilité de 87 % et une spécificité de 80 %.

Le seuil de pente différentielle < à 9 µm/N permet le diagnostic préopératoire de réparation d'un faisceau avec une sensibilité de 67 % et une spécificité de 80 %.

Plus précisément la convergence des deux mesures en faveur d'une réparation partielle a alors montré une valeur prédictive positive de 73,3 % et la convergence des deux mesures (> à 3 mm et > à 9 mm/N) en faveur d'une réparation complète avait une valeur prédictive positive de 97,3 %.

On voit donc qu'avec l'invention il est possible de prévoir une réparation mono faisceau en cas de convergence des informations.

Les informations divergentes correspondent quant à elles à des lésions en continuité ou à des ligaments rebranchés en site non anatomique. Dans ces cas là une réparation plastique classique a pu être effectuée.

En d'autres termes il est ainsi possible, avec l'invention, de programmer une réparation partielle avec une grande sensibilité et spécificité, et ce à partir des données de laxité et de pentes des courbes d'élasticité, la combinaison des deux tests (étirement et pente) permettant d'obtenir de très bonnes valeurs prédictives en cas de convergence.

On va maintenant décrire le fonctionnement de l'appareil mettant en oeuvre la méthode de mesure en référence à la figure 8.

Après positionnement du patient sur l'assise en orientant le dossier de façon à ce qu'il soit installé de façon confortable, puis fixation de sa jambe sur le plateau, et de son pied sur le support de pied (étape 69), on place alors le plateau (étape 70) dans un premier angle avec l'assise par exemple de 30° puis on exerce progressivement une force avec l'organe de poussée sur le mollet tout en détectant le déplacement de la face antéro-supérieure du tibia du patient grâce au capteur de détection du déplacement de façon à obtenir une courbe selon les courbes des figures 3 ou 4 sur l'une puis sur l'autre jambe (étape 71).
On effectue ensuite l'examen et la comparaison des courbes obtenues, à la fois en ce qui concerne le différentiel de laxité, et celui des pentes entre les deux jambes, que l'on compare avec les valeurs seuils, par exemple de 3 mm et 9 µm/N ci-dessus mentionnés, par le biais du calculateur (étape 72).

Cette étape peut également avantageusement comporter une mesure en rotation du pied qui, si elle montre un angle de rotation par exemple selon le degré de liberté 38, dans le plan perpendiculaire à la jambe, supérieur à une valeur seuil déterminée, liée à l'angle α de flexion avec l'assise, par exemple de 15°, pour un angle α de 30°, permet de compléter le diagnostic.

Si nécessaire (test 73), on réitère les étapes précédentes (liaison 74) dans une deuxième position angulaire par exemple 40° puis ensuite 50°, 60°, 70°.

On intègre alors (étape 75) les courbes du type de celles obtenues à la figure 4 avec les angles différents.

Puis on identifie (étape 76) grâce à la différence de comportement des ligaments entre angles différents par exemple entre 30° et 60°) si il s'agit d'un ligament AM ou d'un ligament PL.

Il est ainsi possible d'en déduire (étape 77) le diagnostic de la lésion qui s'affiche par exemple sous forme de message sur l'écran de l'indicateur 40 et ensuite d'effectuer en 78 si il y a lieu, l'opération nécessaire pour rectifier la pathologie.

Dans le mode de réalisation plus particulièrement décrit ci le diagnostic de lésion partielle est effectué selon la grille de mesures successives et/ou simultanées suivantes :
- Mesure à 134N du différentiel d entre genou sain et genou suspect
   Si d < 1mm pas de lésion
   Si 1 < d < 3 mm lésion partielle
   Si d > 3 mm lésion totale
- En cas de risque de lésion (d > 1 mm) mesure du différentiel δ de pentes entre genoux sains et genoux suspects.
   Si 0 < δ < 5 µm/N pas pris en compte
   Si 5 < δ < 9 µm/N lésion partielle
   Si δ > 9 µm/N lésion totale.
- Si lésion partielle, mesures à 30°, 60° et 90° d'inclinaison
   Si d_{60°} > d_{30°} alors lésion AM
   Si d_{60°} < d_{30°} alors lésion PL.

Comme il va de soi et comme il résulte d'ailleurs de ce qui précède la présente invention ne se limite pas aux modes de réalisation plus particulièrement décrits. Elle en embrasse au contraire toutes les variantes et notamment celles où l'appareil comporte deux plateaux de support en parallèle permettant d'effectuer des mesures simultanées des deux jambes l'une saine et l'autre pathologique ce qui accélère le temps et la fiabilité des mesures concernées.

## Revendications

1. Appareil (17) de mesure pour détection d'une lésion du ligament croisé antérieur du genou (18) d'un patient (19), comprenant une assise (20) du patient munie d'un dossier (21) orientable, un plateau (23) support de la jambe (24) du patient (19), mobile en rotation par rapport à l'assise (20), ledit plateau (23) étant muni d'un organe (28) de poussée du mollet (29) du patient (19) et d'un capteur (30) de détection de déplacement de la face antéro-supérieure (31) du tibia du patient (19), lorsque le patient (19) est assis sur l'assise (20), des moyens (32) de fixation de la jambe audit plateau (23) support, des moyens (33) de mesure en continu du déplacement du capteur (30) en fonction de la force exercée par l'organe (28) de poussée sur le mollet (29) et un support (34) du pied dudit patient librement mobile dans les trois degrés de liberté en rotation (36, 37, 38),
**caractérisé en ce que** le plateau (23) support de la jambe est mobile en translation par rapport à l'assise (20) en fonction de l'angle d'inclinaison du plateau par rapport à l'assise (20),
et **en ce que** l'appareil comporte des moyens (40) d'enregistrement des courbes (41) obtenues pour l'une et l'autre des jambes d'un même patient (19) donnant le déplacement du capteur (30) en fonction de la force de poussée dans des inclinaisons différentes du plateau support par rapport à l'assise (20), et des moyens (42) de calcul agencés pour mesurer le différentiel entre ces courbes (41) et en déduire la lésion partielle du ligament antéro-médial et/ou du ligament postéro-latéral.

2. Appareil selon la revendication 1, **caractérisé en ce que** le support (34) du pied est monté libre en translation dans le sens transversal par rapport au plateau (23) support, sa position étant fixe et réglable pour les deux autres degrés de liberté en translation, à savoir en hauteur et dans le sens longitudinal du dit support.

3. Appareil selon l'une quelconque des revendications précédentes, **caractérisé en ce que** le support (34) du pied comporte des moyens capteurs d'au moins un angle de rotation dudit support et **en ce que** les moyens (42) de calcul sont agencés pour mesurer le mouvement de rotation enregistré par ledit capteur induit par le tibia lors de la flexion du plateau (23) support par rapport à l'assise (20).

4. Appareil selon l'une quelconque des revendications précédentes, **caractérisé en ce que** il comporte de plus au moins un capteur/détecteur de la contraction des muscles de la cuisse, et des moyens (42) de traitement pour empêcher la prise en compte des mesures de déplacements et/ou de force de poussée tant que ladite contraction musculaire n'est pas descendue en dessous d'un seuil déterminé.

5. Appareil selon l'une quelconque des revendications précédentes, **caractérisé en ce que** le capteur (30) de détection de déplacement est fixé de façon réglable en hauteur à une potence (63) de support du capteur (30) solidaire du plateau (23) support de la jambe.

6. Appareil selon l'une quelconque des revendications précédentes, **caractérisé en ce que** l'organe (28) de poussée comprend un coussin (43) de soutien du mollet fixé rigidement à une biellette (44) reliée de façon articulée à un système de vérin (46) axial monté parallèle au plateau (23) support et propre à actionner perpendiculairement à sa course ladite biellette articulée, entre une position de repos où la jambe repose librement sur le plateau (23) support et une position où la force exercée sur le mollet (29) atteint au moins 350 N lorsque la jambe (24) est fixée au plateau (23) support.

7. Appareil selon l'une quelconque des revendications précédentes, **caractérisé en ce que** les moyens (32) de fixation de la jambe (24) au plateau (23) support comporte une coque (27, 67) de maintien quadricipital agencée pour être en contact avec le dessus de l'extrémité de la cuisse, rattachée au plateau (23) support par un jeu de deux sangles (68) parallèles semi-rigides réglables en serrage.

## Patentansprüche

1. Messgerät (17) zum Erfassen einer Läsion des vorderen Kniekreuzbandes (18) eines Patienten (19), umfassend eine Patientensitzfläche (20) mit einer ausrichtbaren Rückenlehne (21), eine Platte (23) zum Stützen des Beins (24) des Patienten (19), die in Bezug auf die Sitzfläche (20) drehbeweglich ist, wobei die Platte (23) mit einem Glied (28) zum Schieben der Wade (29) des Patienten (19) versehen ist sowie mit einem Sensor (30) zum Erfassen der Verschiebung der anterosuperioren Seite (31) des Schienbeins des Patienten (19), wenn der Patient (19) auf der Sitzfläche (20) sitzt, Mittel (32) zum Fixieren des Beins an der Stützplatte (23), Mittel (33) für die kontinuierliche Messung der Verschiebung des Sensors (30) in Abhängigkeit von der Kraft, die durch das Schiebeglied (28) gegen die Wade (29) ausgeübt wird, und eine Stütze (34) für den Fuß des Patienten, die in den drei Freiheitsgraden frei drehbeweglich (36, 37, 38) ist,
**dadurch gekennzeichnet,**
**dass** die Platte (23) zum Stützen des Beins in Abhängigkeit von ihrem Neigungswinkel zur Sitzfläche (20) in Bezug auf die Sitzfläche (20) verschieblich ist und das Gerät Mittel (40) für die Aufzeichnung der Kurven (41) aufweist, die für beide Beine ein und desselben Patienten (19) erzielt werden, woraus sich die Verschiebung des Sensors (30) in Abhängigkeit von der Schubkraft bei unterschiedlichen Neigungen der Stützplatte in Bezug auf die Sitzfläche (20) ergibt, und Rechenmittel (42), die angeordnet sind, um den Unterschied zwischen diesen Kurven (41) zu messen und daraus die partielle Läsion des anteromedialen Bandes oder des posterolateralen Bandes abzuleiten.

2. Gerät nach Anspruch 1,
**dadurch gekennzeichnet,**
**dass** die Fußstütze (34) frei verschieblich in Querrichtung in Bezug auf die Stützplatte (23) angebracht ist, wobei ihre Stellung fest und für die zwei weiteren Verschiebungsfreiheitsgrade, und zwar in der Höhe und in Längsrichtung der Stütze, einsteilbar ist.

3. Gerät nach einem der vorhergehenden Ansprüche,
**dadurch gekennzeichnet,**
**dass** die Fußstütze (34) Mittel zur Erfassung mindestens eines Drehwinkels der Stütze aufweist und dass die Rechenmittel (42) angeordnet sind, um die von dem Sensor aufgezeichnete und durch das Schienbein bei der Biegung der Stützplatte (23) in Bezug auf die Sitzfläche (20) induzierte Drehbewegung zu messen.

4. Gerät nach einem der vorhergehenden Ansprüche,
**dadurch gekennzeichnet,**
**dass** es ferner mindestens einen Sensor/Detektor der Anspannung der Oberschenkelmuskel aufweist und Verarbeitungsmittel (42), um die Berücksichtigung der Messungen von Verschiebungen und/oder der Schubkraftmessungen zu verhindern, solange die Muskelanspannung nicht unter einen bestimmten Schwellenwert gefallen ist.

5. Gerät nach einem der vorhergehenden Ansprüche,
**dadurch gekennzeichnet,**
**dass** der Sensor (30) zum Erfassen einer Verschiebung höheneinstellbar an einem Galgen (63) zum Tragen des Sensors (30) befestigt ist, der mit der Platte (23) zum Stützen des Beins verbunden ist.

6. Gerät nach einem der vorhergehenden Ansprüche,
**dadurch gekennzeichnet,**
**dass** das Schiebeglied ein Kissen (43) zum Unterstützen der Wade aufweist, welches starr mit einem Arm (44) verbunden ist, der mit einem axialen Zylindersystem (46) angelenkt verbunden ist, das parallel zur Stützplatte (23) montiert ist und geeignet ist, den angelenkten Arm senkrecht zu seinem Hub zu betätigen zwischen einer Ruhestellung, in der das Bein frei auf der Stützplatte (23) liegt, und einer Stellung, in der die auf die Wade (29) ausgeübte Kraft mindestens 350 N erreicht, wenn das Bein (24) an der Stützplatte (23) fixiert ist.

7. Gerät nach einem beliebigen der vorhergehenden Ansprüche,
**dadurch gekennzeichnet,**
**dass** die Mittel (32) zur Fixierung des Beins (24) an der Stützplatte (23) eine Schale (27, 67) zur Halterung des Quadrizeps aufweisen, die angeordnet ist, um mit dem oberen Bereich des Oberschenkelendes in Berührung zu sein, und die mit der Stützplatte (23) über einen Satz aus zwei parallelen, halbstarren und spannungsregulierbaren Gurten (68) verbunden ist.

## Claims

1. Measuring apparatus (17) for detecting a lesion of the anterior cruciate ligament of the knee (18) of a patient (19), comprising a seat (20) for the patient provided with an adjustable backrest (21), a plate (23) for supporting the leg (24) of the patient (19), movable in rotation in relation to the seat (20), the said plate (23) being provided with a device (28) for applying pressure to the calf (29) of the patient (19) and a sensor (30) for detecting displacement of the anterior superior face (31) of the tibia of the patient (19) when the patient (19) is sitting on the seat (20), means (32) for fixing the leg to the said supporting plate (23), means (33) for continuous measurement of the movement of the sensor (30) as a function of the force exerted by the device (28) for applying pressure to the calf (29) and a support (34) for the foot of the said patient freely movable in the three degrees of freedom in rotation (36, 37, 38),
**characterised in that** the plate (23) for supporting the leg is movable in translation in relation to the seat (20) as a function of the angle of inclination of the plate in relation to the seat (20),
and **in that** the apparatus has means (40) for recording the curves (41) obtained for each of the legs of a same patient (19) giving the movement of the sensor (30) as a function of the pressure force in the different inclinations of the supporting plate in relation to the seat (20), and calculating means (42) arranged to measure the differential between these curves (41) and deduce therefrom a partial lesion of the anterior medial ligament and/or of the posterior lateral ligament.

2. Apparatus according to claim 1, **characterised in that** the support (34) for the foot is mounted free to move by translation in the transverse direction in relation to the supporting plate (23), its position being fixed and adjustable for the two other degrees of freedom in translation, namely vertically and in the longitudinal direction of the said support.

3. Apparatus according to any one of the preceding claims, **characterised in that** the support (34) for the foot has sensing means for at least one angle of rotation of the said support and **in that** the calculating means (42) are arranged to measure the rotational movement recorded by the said sensor induced by the tibia during the flexion of the supporting plate (23) in relation to the seat (20).

4. Apparatus according to any one of the preceding claims, **characterised in that** it additionally comprises at least one sensor/detector for the contraction of the muscles of the thigh, and processing means (42) for preventing implementation of the measurements of movements and/or pressure force applied as long as the said muscular contraction has not fallen below a given threshold.

5. Apparatus according to any one of the preceding claims, **characterised in that** the sensor (30) for detecting displacement is fixed so as to be adjustable in height to a bracket (63) for supporting the sensor (30) secured to the plate (23) for supporting the leg.

6. Apparatus according to any one of the preceding claims, **characterised in that** the device (28) for applying pressure comprises a cushion (43) for supporting the calf fixed rigidly to a rod (44) connected in articulated fashion to an axial jack system (46) mounted parallel to the supporting plate (23) and capable of actuating the said articulated rod perpendicularly to its travel, between a resting position in which the leg rests freely on the supporting plate (23) and a position in which the force exerted on the calf (29) reaches at least 350 N when the leg (24) is fixed to the supporting plate (23).

7. Apparatus according to any one of the preceding claims, **characterised in that** the means (32) for fixing the leg (24) to the supporting plate (23) has a quadricipital holding shell (27, 67) arranged to be in contact with the upper side of the end of the thigh, attached to the supporting plate (23) by a set of two semi-rigid parallel straps (68) which are adjustable for tightness.
